# EUROPÄISCHE PATENTSCHRIFT

(11) **EP 1 493 423 B1**
(45) Veröffentlichungstag und Bekanntmachung des Hinweises auf die Patenterteilung: **14.06.2006**
(21) Anmeldenummer: 03013799.6
(22) Anmeldetag: 18.06.2003
(51) Int. Cl.: A61K 8/00

(54) **Haarbehandlungsmittel und Haarnachbehandlungsmittel zum Schutz vor Schäden durch chemische Behandlung und zur Reparatur bereits geschädigter Haare enthaltend als Wirksubstanz Alkylguanidin-Verbindungen**
Composition for hair treatment and for the after-treatment of hair for protection against damage by chemical treatment and for the repair of pre-existing damaged hair, comprising alkyl-guanidinium derivatives as active agent
Composition pour le traitement et pour le post-traitement des cheveux afin de les protéger contre les dommages induits par traitement chimique et pour la réparation des dommages pré-existants des cheveux, contenant comme agent actif des composés à base d'alkyle de guanidine

(43) Veröffentlichungstag der Anmeldung: 05.01.2005
(73) Patentinhaber: Goldschmidt GmbH, 45127 Essen (DE)
(72) Erfinder: Pascaly, Matthias, Dr., 45131 Essen (DE); Lersch, Peter, Dr., 46537 Dinslaken (DE); Flintrop, Nicole, 47259 Duisburg (DE); Muss, Peter, 45359 Essen (DE); Maczkiewitz, Ursula, 45219 Essen (DE); Grüning, Burghard, Dr., 45134 Essen (DE); Weitemeyer, Christian, 45134 Essen (DE)

(56) Entgegenhaltungen:
- EP-A- 1 285 647
- US-A1- 2002 172 655
- PATENT ABSTRACTS OF JAPAN vol. 1999, no. 05, 31. Mai 1999 (1999-05-31) & JP 11 035546 A (KAO CORP), 9. Februar 1999 (1999-02-09)
- PATENT ABSTRACTS OF JAPAN vol. 1999, no. 05, 31. Mai 1999 (1999-05-31) & JP 11 035425 A (KAO CORP), 9. Februar 1999 (1999-02-09)
- PATENT ABSTRACTS OF JAPAN vol. 1999, no. 05, 31. Mai 1999 (1999-05-31) & JP 11 035424 A (KAO CORP), 9. Februar 1999 (1999-02-09)
- PATENT ABSTRACTS OF JAPAN vol. 016, no. 234 (C-0945), 29. Mai 1992 (1992-05-29) & JP 04 049222 A (LION CORP), 18. Februar 1992 (1992-02-18)
- PATENT ABSTRACTS OF JAPAN vol. 1997, no. 11, 28. November 1997 (1997-11-28) & JP 09 194333 A (KAO CORP), 29. Juli 1997 (1997-07-29)
- PATENT ABSTRACTS OF JAPAN vol. 2002, no. 05, 3. Mai 2002 (2002-05-03) & JP 2002 029936 A (LION CORP), 29. Januar 2002 (2002-01-29)

## Beschreibung

Menschliches Haar ist täglich den verschiedensten Einflüssen ausgesetzt. Neben mechanischen Beanspruchungen durch Bürsten, Kämmen, Hochstecken oder Zurückbinden, werden die Haare auch durch Umwelteinflüsse wie z.B. starke UV-Strahlung, Kälte, Wind und Wasser angegriffen. Auch der physiologische Status (z.B. Alter, Gesundheit) der jeweiligen Person beeinflusst die Schädigung der keratinischen Fasern.

Insbesondere aber auch die Behandlung mit chemischen Mitteln verändert Struktur und Oberflächeneigenschaften der Haare. Methoden wie z.B. dass Dauerwellen, Bleichen, Färben, Tönen, Glätten usw., aber auch häufiges Waschen mit aggressiven Tensiden tragen dazu bei, dass mehr oder weniger starke Schäden an der Haarstruktur verursacht werden. So wird z.B. bei einer Dauerwelle sowohl die Cortex als auch die Cuticula des Haares angegriffen. Die Disulfid-Brücken des Cystins werden durch den Reduktionsschritt aufgebrochen und im anschließenden Oxidationsschritt zum Teil zu Cysteinsäure oxidiert.

Beim Bleichen wird nicht nur das Melanin zerstört, sondern es werden außerdem ca. 15 bis 25 Gew.-% der Disulfid-Bindungen des Cystins bei einer milden Bleiche oxidiert. Bei einer exzessiven Bleichung können es sogar bis zu 45 Gew.-% sein (K. F. de Polo, A Short Textbook of Cosmetology, 2000, Verlag für chemische Industrie, H. Ziolkowsky GmbH).

So ergeben sich aus den chemischen Behandlungen, dem häufigen Waschen oder der UV-Bestrahlung nachteilige mechanische Eigenschaften für das Haar. Es wird spröde, trocken, glanzlos, porös und schlecht kämmbar. Außerdem verliert es Feuchtigkeit, Elastizität und vor allem mechanische Widerstandsfähigkeit und Reißfestigkeit. Dies zeigt sich in einer signifikanten Abnahme der Zug-Dehnungskräfte und der Reißkräfte bei nassem Haar. Außerdem ist es gegenüber einer weiteren Schädigung durch Chemikalien, Tenside und Umwelteinflüsse weniger widerstandsfähig als gesundes Haar.

Für die Reparatur derart geschädigter Haare gibt es spezielle Zubereitungen, wie z.B. Haarspülungen, Haarkuren, Shampoos, Leave-in Konditionierer usw., die jedoch vor allem die Kämmbarkeit, den Griff und den Glanz geschädigter Haare verbessern können. Derartige handelsübliche Haarpflegemittel enthalten hauptsächlich kationische Tenside auf Alkylammonium-Basis, Polymere, Wachse bzw. Öle. Die Wirksamkeit dieser Verbindungen lässt sich auf eine elektrostatische Wechselwirkung der kationischen Quat-Gruppen bzw. auf eine Hydrophobisierung der Haaroberfläche zurückführen. Eine (bio)chemische Reparatur des Haares wird dadurch jedoch nicht erreicht.

Dem speziellen Problem, dass bei Haaren durch Schädigung die mechanische Widerstandsfähigkeit stark reduziert wird, wird schon seit langem große Aufmerksamkeit geschenkt.

In diesem Zusammenhang ist beispielsweise der Einsatz von Kreatin bekannt. In DE-A-101 14 561 und DE-A-101 196 08 wird die Verwendung von Kreatin-Verbindungen in Mitteln zur Härtung, Stärkung, Restrukturierung oder Erhöhung von Glanz, Volumen oder Kämmbarkeit von keratinischen Fasern, insbesondere menschlichen Haaren beschrieben.

Ein Nachteil bei der Verwendung von Kreatin ist die Tatsache, dass Kreatin nur über die wässrige Phase formuliert werden kann und es sich in wässrigen Lösungen immer in einem Gleichgewicht mit Kreatinin befindet und somit als aktive Substanz für das Haar nicht mehr zur Verfügung steht.

Vor diesem Hintergrund ist es von großer Wichtigkeit weitere neue Substanzen zu identifizieren, die eine dem Kreatin ähnliche Struktur aufweisen und/oder eine ähnliche physiologische Wirkung erzielen oder auch die Wirkung von Kreatin unterstützen und verstärken.

Es besteht daher also weiterhin ein Bedarf an vielseitig einsetzbaren Wirkstoffen für Haarbehandlungsmittel und Haarnachbehandlungsmittel, die die mechanische Widerstandsfähigkeit des Haares verbessern, das Haar vor weiterer Schädigung der Haarstruktur schützen und die bereits verursachten strukturellen Schädigungen des Haares, hervorgerufen durch Umwelteinflüsse sowie form- und farbgebende Behandlungen eingetreten sind, zu minimieren.

Es ist eine Aufgabe der Erfindung, einen solchen Wirkstoff zur Verfügung zu stellen, der in der Lage ist, sowohl die mechanische Widerstandsfähigkeit von geschädigten Haaren zu verbessern, als auch das Haar vor Schädigungen durch eine chemische Behandlung oder exogenen Faktoren wie Rauchen, Smog, reaktive Sauerstoffspecies, freie Radikale und insbesondere lichtbedingten Schädigungen zu schützen.

Es wurde nun überraschenderweise gefunden, dass einfache Alkylguanidin-Verbindungen und deren Säure-Konjugate in Zubereitungen zur Behandlung und Nachbehandlung der Haare alle diese gewünschten Kriterien erfüllen.

Ein Gegenstand der Erfindung ist daher die Verwendung von Alkylguanidinverbindungen der allgemeinen Formeln (I) und/oder (II) 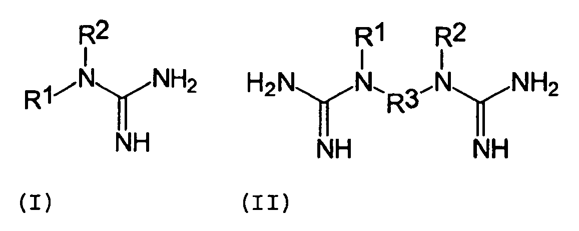 und/oder deren Salze oder Hydrate, in denen
- R¹, R² = a): unabhängig voneinander H, ein gegebenenfalls verzweigter, gegebenenfalls Doppelbindungen enthaltender Kohlenwasserstoffrest, Hydroxyalkyl-, Alkyloxy-, Carboxyalkylreste, mit 2 bis 30 C-Atomen, vorzugsweise 4 bis 22, insbesondere 8 bis 12 C-Atomen, oder
- R¹, R² = b): wobei die Reste auch ali- oder heterocyclische Komponenten, gesättigt, ungesättigt oder aromatisch, mit einer Ringgröße von 3 bis 10 Atomen, vorzugsweise von 4 bis 6 Atomen, besitzen können, welche weitere, gesättigte oder ungesättigte Kohlenwasserstoffsubstituenten mit 1 bis 30 C-Atomen, vorzugsweise 4 bis 22 C-Atomen, tragen können, oder
- R¹,R² = c): Alkylamidoalkylen- oder Alkylesteralkylenreste, die weitere unter a) und b) genannte Strukturelemente enthalten können,
und die Strukturelemente a), b), c) untereinander und miteinander kombiniert werden können,

- R³: Alkylen, gegebenenfalls verzweigter, gegebenenfalls Doppelbindungen oder ali- oder heterocyclische Komponenten, gesättigt, ungesättigt oder aromatisch, mit einer Ringgröße von 3 bis 10 Atomen, vorzugsweise mit einer Ringgröße von 4 bis 6 Atomen, enthaltender Kohlenwasserstoffrest mit 1 bis 30 C-Atomen, vorzugsweise 4 bis 22 C-Atomen oder in der R¹ und R² in dem Element -N(R¹)-R³-(R²)N- einen 5- bis 8-gliedrigen Ring bilden kann, enthalten
als Wirksubstanzen zur Herstellung eines Haarbehandlungsmittels/Haarnachbehandlungsmittels zur Verhütung von Schäden durch chemische Behandlungsmittel und zur Reparatur bereits geschädigter Haare.

Die erfindungsgemäß verwendeten oder mitverwendeten Alkylguanidine, besitzen sowohl eine gute Stabilität als auch eine gute Formulierbarkeit, rufen bereits in geringen Einsatzkonzentrationen eine deutliche Wirkung hervor, sind nicht toxisch, natürlichen Ursprungs oder naturidentisch, werden sehr gut vom Haar und der Kopfhaut toleriert, weisen eine hohe Verträglichkeit mit anderen Inhaltsstoffen auf und lassen sich problemlos in Haarbehandlungsmittel einarbeiten. Zusätzlich können sie noch eine leicht antimikrobielle Wirkung aufweisen.

Die Herstellung von Alkylguanidinen ist in der DE-C-506 282 beschrieben. In dem Verfahren werden Alkylamine in einer alkoholischen Lösung mit Cyanamid in Anwesenheit einer Protonensäure guanidyliert. So werden die Produkte als kristalline Salze erhalten.

In der DE-A-195 27 313 werden Verfahren zur Herstellung von Guanidin-Derivaten und deren Verwendung als Hautkosmetika beschrieben. Dies beinhaltet (Poly)Alkylguanidine und Alkoxyguanidine, die bei Verwendung ein gutes Hautgefühl hervorrufen und eine feuchtigkeitsspendende und Keratin-weichmachende Wirkung besitzen.

In der JP-A-2-47866 werden ebenfalls Hautkosmetika beschrieben, welche einen exzellenten Pflegeeffekt aufweisen und Kreatin und/oder Kreatinin in Verbindung mit einem weiteren pharmazeutischen Wirkstoff und/oder einer bioaktiven Substanz beinhalten.

In der JP-A-5-194150 wird die Verwendung von C₆₋₂₄-Alkylguanidinen in Kombination mit Fettalkoholen als Haarkonditioniermittel beschrieben.

Patent Abstracts of Japan, vol. 1999(5), 31/05/1999 & JP-A-11035546 (KAO CORP), 9/02/1999, beschreibt Haarpflegemittel auf Basis von Guanidinderivaten, mit der Wirkung "having a good touch of use, capable of imparting flexibility to hair".

Gemäß Patent Abstracts of Japan, vol. 1999(5), 31/05/1999 & JP-A-11035425 (KAO CORP), 9/02/1999, sowie Patent Abstracts of Japan, vol. 1999(5), 31/05/1999 & JP-A-11035424 (KAO CORP), 9/02/1999 werden Haarkosmetika enthaltend Guanidinverbindungen umfasst, welche "good in feeling when applied, and capable of imparting the hair after dried with excellent wiriness" sind.

Im Patent Abstracts of Japan, vol. 1999(5), 31/05/1999 & JP-A-11035424 (KAO CORP), 9/02/1999, werden Guanidinverbindungen verwendet mit der Wirkung: "giving excellent hair conditioning property".

Der Patent Abstracts of Japan, vol. 1997(11),28/11/1997 & JP-A-09194333 (KAO CORP), 29/07/1997, offenbart Haarpflegemittel enthaltend Guanidinverbindungen "having excellent use feeling and capable of imparting flexibility to hair after drying".

Der Patent Abstracts of Japan, vol. 2002(5),3/05/2002 & JP-A-2002029936 (LION CORP), 29/01/2002, wiederum offenbart guanidin(salz)-haltige Verbindungen in Haarkosmetika, welche "give excellent gloss to the hair after being dried and no dryness and looseness".

Die EP-A-1 285 647, offenbart ebenfalls haarkosmetische Produkte enthaltend Guanidinverbindungen in Kombination mit N-alpha-acylarginderivaten.

In der US-2002/0172655 werden guanidin-haltige haarkosmetische Behandlungsmittel offenbart, die eine gute Haftung auf dem Haar haben und zur Formgebung des Haares genutzt werden können.

Die Verwendung von Alkylguanidin-Derivaten ist nach bekanntem Stand der Technik also beschränkt auf die Anwendung in oder als Konditioniermittel für oberflächliche Behandlungen der Haare und in hautkosmetischen Formulierungen. Über die überraschenden Eigenschaften dieser Verbindungsklasse zur Stärkung und Schutz geschädigter Haare und deren Reparatur ist bislang nichts zu finden.

Als besonders geeignet im Sinne der vorliegenden Erfindung haben sich Alkylguanidine der allgemeinen Formel (I) und/oder deren Salze und/oder deren Hydrate erwiesen und werden daher bevorzugt.

Zur Salzbildung geeignet sind grundsätzlich alle kosmetisch unbedenklichen anorganischen oder organischen ein- oder mehrbasischen Säuren wie beispielsweise Ameisensäure, Essigsäure, Propionsäure, Heptansäure, Caprylsäure, Nonansäure, Caprinsäure, Undecansäure, Laurinsäure, Myristinsäure, Palmitinsäure, Stearinsäure, Arachinsäure, Behensäure, Cyclopentancarbonsäure, Cyclohexancarbonsäure, Acrylsäure, Methacrylsäure, Vinylessigsäure, Crotonsäure, 2-/3-/4-Pentensäure, 2-/3-/4-/5-Hexensäure, Lauroleinsäure, Myristoleinsäure, Palmitoleinsäure, Ölsäure, Gadoleinsäure, Sorbinsäure, Linolsäure, Linolensäure, Pivalinsäure, Ethoxyessigsäure, Phenylessigsäure, Milchsäure, 2-Ethylhexansäure, Oxalsäure, Glycolsäure, Äpfelsäure, Malonsäure, Bernsteinsäure, Weinsäure, Glutarsäure, Zitronensäure, Adipinsäure, Pimelinsäure, Korksäure, Azelainsäure, Sebacinsäure, Benzoesäure, o-/m-/p-Tolylsäure, Salicylsäure, 3-/4-Hydroxybenzoesäure, Phthalsäuren, oder deren ganz oder teilweise hydrierten Derivate wie Hexahydro- oder Tetrahydrophthalsäure, Kohlensäure, Phosphorsäure, Salzsäure, Schwefelsäure und deren Gemische, sind sehr gut geeignet, insbesondere Milchsäure, Weinsäure, Essigsäure und Salzsäure. Dabei ist es im Sinne der vorliegenden Erfindung auch möglich, sowohl geeignete Guanidin-Derivate untereinander in Mischungen zu verwenden als auch Mischsalze.

Als erfindungsgemäße, kosmetische Zubereitungen zur Nachbehandlung, Formgebung und Pflege der Haare werden vor allem solche Haarbehandlungsmittel verstanden, die nach einer chemischen Behandlung der Haare verwendet werden (Haarnachbehandlungsmittel) und chemische Haarbehandlungsmittel, durch die die Haarstruktur geschädigt wird und bei denen die Schädigung durch den Zusatz von Alkylguanidinverbindungen minimiert werden kann (Haarbehandlungsmittel).

Alkylguanidine können dabei generell in einer Konzentration von 0,01 bis 10,0 Gew.-%, bevorzugt in einer Konzentration von 0,1 bis 5,0 Gew.-%, insbesondere in einer Konzentration von 0,1 bis 2,0 Gew.-%, enthalten sein.

Bei den Haarnachbehandlungsmitteln handelt es sich z.B. um Haarspülungen, Haarkuren, Avivagemittel, Leave-in Konditionierer, Haarshampoos, two-in-one-Shampoos, Festigerformulierungen wie Schaumfestiger, Haarsprays oder Fönlotionen, Haarwässer, Haarspitzenfluids. Sie können als Gel, Emulsion, Lösung, Aerosolspray oder -schaum, Nonaerosolspray oder -schaum vorliegen.

Die erfindungsgemäßen kosmetischen Zubereitungen zur Behandlung der Haare nach einer chemischen Behandlung weisen einen pH-Wert von 3 bis 7 auf und enthalten daher bevorzugt eine dafür geeignete wasserlösliche Säure oder ein Puffergemisch, das diesen pH-Wert stabilisiert.

Die erfindungsgemäßen, kosmetischen Zubereitungen zur Behandlung der Haare nach einer chemischen Behandlung können neben Alkylguanidin-Verbindungen noch weitere Komponenten enthalten, die für den jeweiligen Anwendungszweck vorteilhaft und/oder üblich sind.

So können Shampoos z.B. 3 bis 30 Gew.-% schäumende anionische, zwitterionische, ampholytische und nichtionische Tenside enthalten. Haarspülungen und Haarkuren enthalten 0 bis 10 Gew.-%, vorzugsweise 0,5 bis 5 Gew.-%, Emulgatoren, 0 bis 10 Gew.-%, vorzugsweise 0,5 bis 5 Gew.-%, Konsistenzgeber und 0 bis 20 Gew.-% kosmetische Öle pflanzlichen und synthetischen Ursprungs, Emollients, Vitaminpräparate und Proteine. Shampoos, Haarspülungen, Haarkuren und Avivagemittel enthalten außerdem bevorzugt 0 bis 8 Gew.-%, vorzugsweise 0,1 bis 5 Gew.-%, kationische Tenside und wasserlösliche Polymere mit quaternären Ammoniumgruppen zur Verringerung der statischen Aufladbarkeit und zur Verbesserung von Kämmbarkeit, Griff und Glanz.

Bei den kationischen Tensiden handelt es sich in der Regel um
- quaternäre Ammoniumverbindungen, wie z.B. Alkyltrimethylammoniumsalze, Dialkyldimethylammoniumsalze, Trialkylmethylammoniumsalze und Imidazolinium-Verbindungen. Die langen Alkylketten bestehen aus einer Kohlenstoffkette mit 10 bis 22 C-Atomen, die Gegenionen zum quaternären Stickstoff sind z.B. Halogenide, Sulfat, Acetat, Lactat, Glycolat, Nitrat oder Phosphat. Produkte findet man unter der Bezeichnung Varisoft® 300, 432 CG, 442-100 P, BT 85 von Goldschmidt Rewo, Dehyquart® A von Henkel im Handel;
- Esterquats, wie sie unter der Bezeichnung Dehyquart® F75 von Henkel oder Armocare® VGH-70 von Akzo vertrieben werden;
- Alkylamidoquats, wie sie z.B. unter der Bezeichnung Varisoft® PATC und RTM 50 von Goldschmidt Rewo im Handel sind.

Bei den wasserlöslichen Polymeren mit quaternären Ammoniumgruppen handelt es sich z.B. um
- kationische Cellulosederivate, wie sie unter der Bezeichnung Celquat® H 100 und L 200 von National Starch oder Polymer JR® 400 von Amerchol im Handel erhältlich sind,
- polymere Dimethyldiallylammoniumsalze und deren Copolymere mit Estern und Amiden von Acrylsäure und Methacrylsäure. Die unter der Bezeichnung Merquat® 100 oder Merquat® 550 von Calgon im Handel erhältlichen Produkte sind Beispiele für solche kationischen Polymere,
- Copolymere des Vinylpyrrolidons mit quaternierten Derivaten des Dialkylaminoacrylats- und -methacrylates. Solche Verbindungen sind unter der Bezeichnung Gafquat® 735 und Gafquat® 744 von ISP im Handel erhältlich,
- Vinylpyrrolidon-Vinylimidazoliummethochlorid-Copolymere, wie sie unter der Bezeichnung Luviquat® FC 370, FC 550, FC 905 und HM-552 von BASF angeboten werden,
- quaternierter Polyvinylalkohol,
- quaternierte Proteinhydrolysate tierischen oder pflanzlichen Ursprungs auf Basis Keratin, Collagen, Elastin, Weizen, Reis, Soja, Milch, Seide, Mais. Solche Produkte werden z.B. unter der Bezeichnung Croquat® Wheat und Silk von Croda, Promois® W-32CAQ, Silk CAQ, WG CAQ von Seiwa Kasei oder Quat-Coll® CDMA von Brooks vertrieben,
- Guarhydroxypropyltrimethylammoniumchlorid,
- aminofunktionelle Polydimethylsiloxane oder hydroxylaminomodifizierte Silicone, wie die Handelsprodukte ABIL® Quat 3272 und ABIL® Quat 3474 von Goldschmidt, Dow Corning® 929 Emulsion, Dow Corning® 939 von Dow Corning.

Festigerformulierungen sowie andere Haarstyling-Zubereitungen enthalten üblicherweise 0,1 bis 5 Gew.-% filmbildende, in wässrigen oder wässrig-alkoholischen Medien lösliche Polymerisate, gegebenenfalls gemeinsam mit kationischen Tensiden oder kationischen Polymeren. Beispiele für Filmbildner sind Homopolymere des Vinylpyrrolidons, Homopolymere des N-Vinylformamids, Copolymerisate aus Vinylpyrrolidon und Vinylacetat, Terpolymere aus Vinylpyrrolidon Vinylacetat und Vinylpropionat, Polyacrylamide, Polyvinylalkohole, hochmolekulares Polyethylenglykol oder hochmolekulare Copolymere von Ethylenglykol mit Propylenglykol, Chitosan. Diese Produkte findet man unter der Bezeichnung Luviskol® K30, K60, K80, VA37E von BASF oder PVP/VA E335 und PVP K30 von ISP im Handel.

Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Eine typische Formulierung für eine Haarspülung/Haarkur enthält beispielsweise:
a) 0,1 bis 2 Gew.-% mindestens eine der Verbindungen der allgemeinen Formel (I) und/oder (II),
b) 0,1 bis 5 Gew.-% an Emulgator,
c) 0,1 bis 5 Gew.-% an Konsistenzgeber,
d) 0,1 bis 5 Gew.-% an kationischen Tensiden und/oder wasserlösliche Polymere mit quaternären Ammoniumgruppen,
e) 0 bis 10 Gew.-% an sonstigen kosmetischen Wirkstoffen, Konservierungsmittel sowie übliche Zusatz- und Hilfsstoffe,
f) ad 100 Gew.-% Wasser.

Ein typische Formulierung für ein Haarshampoo enthält beispielsweise:
a) 0,1 bis 2 Gew.-% mindestens eine der Verbindungen der allgemeinen Formel (I) und/oder (II),
b) 3 bis 30 Gew.-% schäumende anionische, amphotere, ampholytische oder nichtionische Tenside,
c) 0,1 bis 5,0 Gew.-% kationische Tenside und/oder wasserlösliche Polymere mit quaternären Ammoniumgruppen,
d) 0,1 bis 6,0 Gew.-% Verdicker,
e) 0 bis 10 Gew.-% an sonstigen kosmetischen Wirkstoffen, Trübungsmitteln, Lösungsmitteln sowie übliche Zusatz- und Hilfsstoffe,
f) ad 100 Gew.-% Wasser.

Aufgrund ihrer milden mikrobiziden Wirkung können die Verbindungen der allgemeinen Formel (I) und/oder (II) auch als Wirkstoffe in milden Antischuppenformulierungen verwendet oder mitverwendet werden.

Bei den erfindungsgemäßen Zubereitungen zur chemischen Haarbehandlung handelt es sich um Mittel zur dauerhaften Verformung der Haare wie Dauerwell- und Fixiermittel oder Haarglättungsmittel, farbverändernde Mittel wie Blondiermittel, Oxidationsfärbemittel und Tönungsmittel und -shampoos auf Basis direktziehender Farbstoffe.

Die erfindungsgemäßen Zubereitungen zur chemischen Behandlung der Haare enthalten neben Alkylguanidin-Verbindungen und dessen Derivaten noch weitere Komponenten, die üblicherweise für die jeweilige Anwendung eingesetzt werden.

Dies sind bei einer Dauerwelllösung z.B. 1 bis 10 Gew.-% Thioglykolsäure, Thioglykolsäuresalze oder -ester. Dauerwell-Fixiermittel oder Blondiermittel enthalten bevorzugt 2 bis 10 Gew.-% Oxidationsmittel, wie z.B. Kaliumbromat, Natriumbromat oder Wasserstoffperoxid. Haarglättungsmittel basieren auf dem Einsatz starker Basen oder auf Reduktionsmitteln wie z.B. Thioglycolsäuresalzen. Haarfärbemittel enthalten direktziehende Haarfärbemittel oder Oxidationsfarbstoff-Vorprodukte.

Schließlich können die erfindgsgemäßen Zubereitungen weitere kosmetische Hilfs- und Zusatzstoffe, die in solchen Zubereitungen üblich sind, enthalten. Solche Hilfstoffe sind z.B. Lösungsvermittler wie Ethanol, Isopropanol, Ehtylenglykol, Propylenglykol, Glycerin und Diethylenglykol, Komplexbildner wie EDTA, NTA, β-Alanindiessigsäure und Phosphonsäure, Konservierungsmittel, Antioxidantien, Duftstoffe, Farbstoffe zum Anfärben der kosmetischen Zubereitung, Trübungsmittel wie Latex, Styrol/PVP- und Styrol-Acrylamid-Copolymere, Perlglanzmittel wie Ethylenglykolmono- und -distearat und PEG-3-distearat, Pigmente, Lichtschutzmittel, Verdickungsmittel oder Treibmittel.

Die Alkylguanidin-Verbindungen können in den erfindungsgemäßen, kosmetischen Zubereitungen auch mit anderen haarkosmetischen Wirkstoffen (active ingredients), wie z.B. Ceramiden, Pseudoceramiden, Proteinhydryolysaten pflanzlichen oder tierischen Ursprungs auf Basis Keratin, Collagen, Elastin, Weizen, Reis, Soja, Milch, Seide, Mais, Antischuppenwirkstoffen wie Piroctone Olamine, Zink Omadine und Climbazol, Sebostatika, Vitaminen, Panthenol, Pyrrolidoncarbonsäure, Bisabolol, Pflanzenextrakten kombiniert werden.

Die Herstellung der erfindungsgemäßen Haarbehandlungsmittel erfolgt in der üblichen Weise, wobei die Alkylguanidinverbindungen sowohl in der wässrigen als auch in der Ölphase gelöst werden. Die Einstellung des pH-Wertes erfolgt bevorzugt zuletzt durch Zugabe der dafür vorgesehenen Säure und/oder des Puffergemisches.

Im folgenden Text werden einige Herstellungsbeispiele und Rezepturen angeführt. Diese veranschaulichen den Gegenstand der Erfindung und beschränken diesen nicht.

### Herstellungsbeispiele:

Darstellung von Octylguanidiniumacetat und C₁₆₋₂₂-Alkylguanidiniumacetat:

30,7 g Octylamin bzw. 70,8 g C₁₆₋₂₂-Alkylamin (0,2375 mol) werden unter Rühren bei erhöhter Temperatur in 30 ml n-Butanol gelöst. Während der Aufwärmphase werden 14,2 g Essigsäure (0,2365 mol) zugegeben. Nach Erreichen der Reaktionstemperatur von 90 °C wird über einen Zeitraum von 3 h eine Lösung von 10,0 g Cyanamid (0,2379 mol) in Butanol (ca. 60 ml) zugetropft und der Ansatz bei 90 °C für 3 h weitergerührt. Nach dem Abkühlen wird das Lösungsmittel am Rotationsverdampfer unter vermindertem Druck abgezogen. Das Produkt wird mit Aceton aufgeschlämmt, abfiltriert und mit Diethylether gewaschen. Das Endprodukt liegt als kristallines farbloses Pulver vor.

Octylguanidiniumacetat: ¹³C-NMR, 100 MHz, CD₃OD, 25 °C: δ = 181,0 (1C, COOH_{AcOH}), 159,3 (1C, C_{Guanidiniumgr.}), 42,9 (1C, CH₂), 33,4 (1C, CH₂), 30,8 (1C, CH₂), 30,4 (1C, CH₂), 28,2 (1C, CH₂), 24,8 (1C, CH₂), 24,1 (1C, CH_{3,AcOH}), 14,9 (1C, CH₃); MALDI-TOF[m/z]: 172,2 (M+H⁺) .

C₁₆₋₂₂-Alkylguanidiniumacetat: ¹³C-NMR, 100 MHz, CD₃OD, 50 C: δ = 178,5 (1C, COOH_{AcOH}), 157,1 (1C, C_{Guanidingr.}), 40,6 (1C, CH₂), 31,2 (1C, CH₂), 28, 9 (1C, CH₂), 28,5 (1C, CH₂), 28, 5 (1C, CH₂), 28,1 (1C, CH₂), 25,9 (1C, CH₂), 22,5 (1C, CH₂), 21,8 (1C, CH_{3,AcOH}), 12,6 (1C, CH₃); MALDI-TOF[m/z]: 284,2 (C₁₆₋Guanidin+H⁺), 312,2 (C₁₈-Guanidin+H⁺), 340, 2 (C₂₀-Guanidin+H⁺), 368,3 (C₂₂-Guanidin+H⁺).

### Darstellung von Octylguanidiniumchlorid:

15,4 g Octylamin (0,1194 mol) und 20 ml n-Butanol werden vorgelegt und unter Rühren auf 90 °C erwärmt. Während der Aufwärmphase werden 11,0 g Salzsäure (37 %; 0,1137 mol) zugegeben. Nach Erreichen der Reaktionstemperatur von 90 °C wird über einen Zeitraum von 3 h eine Lösung von 5,0 g Cyanamid (0,1308 mol) in Butanol (ca. 60 ml) zugetropft und der Ansatz bei 90 °C für 4 h weitergerührt. Nach dem Abkühlen wird das Lösungsmittel am Rotationsverdampfer unter vermindertem Druck abgezogen. Das Produkt wird in H₂O gelöst und mit NaCl-Lösung abgetrennt. Trocknung des Produktes erfolgt durch Zugabe von Ethanol und anschließender Destillation. Das Endprodukt ist eine hochviskose farblose Flüssigkeit.
¹³C-NMR, 100 MHz, CD₃OD, 25 °C: δ = 158,6 (1C, C_{Guanidiniumgr.}), 42,9 (1C, CH₂), 42,8 (1C, CH₂) , 33,0 (1C, CH₂) , 30,4 (1C, CH₂), 29,9 (1C, CH₂), 27,8 (1C, CH₂), 23,8 (1C, CH₂), 14,8 (1C, CH₃) ; MALDI-TOF [m/z] : 172, 2 (M+H⁺) , 192, 9 (M+Na⁺) .

### Darstellung von Octylguanidiniumlactat:

In einem Mehrhalsrundkolben werden 15,4 g Octylamin (0,1194 mol) und 20 ml n-Butanol vorgelegt und unter Rühren auf 90 °C erwärmt. Während der Aufwärmphase wird 10,2 g Milchsäure (0,1137 mol) zugegeben. Nach Erreichen der Reaktionstemperatur von 90 °C wird über einen Zeitraum von 3 h eine Lösung von 5,0 g Cyanamid (0,1194 mol) in Butanol (ca. 60 ml) zugetropft und der Ansatz bei 90 °C für 3 h weitergerührt. Nach dem Abkühlen wird das Lösungsmittel am Rotationsverdampfer unter vermindertem Druck abgezogen. Das Produkt wird mit Aceton aufgeschlämmt, abfiltriert und mit Diethylether gewaschen. Das Endprodukt liegt als kristallines farbloses Pulver vor.
¹³C-NMR, 100 MHz, CD₃OD, 20 °C: δ = 182,4 (1C, COOH_{Milchs.}), 158,7 (1C, C_{Guanidiniumgr.}), 69,6 (1C, COH_{Milchs.}), 42,5 (1C, CH₂), 33,0 (1C, CH₂), 30, 34 (1C, CH₂), 30,0 (1C, CH₂), 27,8 (1C, CH₂), 23,7 (1C, CH₂), 21,7 (1C, CH_{3,Milchs.}), 14,5 (CH₃) ; MALDI-TOF[m/z]: 172,1 (M+H⁺) .

### Darstellung von Hexan-1,6-diguanidiniumacetat:

36,27 g (0,312 mol) 1,6 Diaminohexanm, 37,49 g (0,624 mol) Essigsäure und 180 ml n-Butanol werden vorgelegt und auf 90 °C erwärmt. Das Cyanamid wird in 80 ml 1-Butanol gelöst und bei 90 °C über 3 Stunden kontinuierlich zugetropft. Nach Beendigung der Zugabe lässt man 4 Stunden bei 90 °C nachreagieren. Am Rotationsverdampfer wird das Lösungsmittel abgezogen. Danach kristallisiert man aus Wasser um, wäscht den Rückstand 2 mal mit je 50 ml Aceton und trocknet das Produkt bei 60 °C im Vakuum. Das Endprodukt wird als kristallines hellbeiges Pulver erhalten.
¹³C-NMR, 100 MHz, D₂O, 20 °C: δ = 181,1 (2C, COOH_{AcOH}), 156,6 (2C, C_{Guanidiniumgr.}), 40,9 (2C, CH₂), 27,6 (2C, CH₂), 25,3 (2C, CH₂), 23,2 (2C, CH_{3,AcOH}); MALDI-TOF[m/z]: 201,2 (M+H⁺).

### Darstellung von N,N-Dibutylguanidiniumacetat:

55,86 g (0.432 mol) Dibutylamin, 25,96 g (0,432 mol) Essigsäure und 100 ml 1-Butanol werden vorgelegt und auf 90 °C erwärmt. Das Cyanamid wird in 60 ml n-Butanol gelöst und bei 90 °C über 3 Stunden kontinuierlich zugetropft. Nach Beendigung der Zugabe lässt man 4 Stunden bei 90 °C nachreagieren. Bei 76 °C wird das ausgefallene Salz abfiltriert und 2 mal mit je 50 ml Aceton gewaschen. Am Rotationsverdampfer wird das Produkt bei 60 °C im Vakuum getrocknet, und das Endprodukt wird als farblose kristalline Substanz erhalten.
¹³C-NMR, 100 MHz, Ethanol-d6, 20 °C: δ = 178,8 (1C, COOH_{AcOH}), 155,9 (1C, C_{Guanidiniumgr.}), 48,1 (2C, CH₂), 29,0 (2C, CH₂) , 23,3 (1C, CH_{3,AcOH}), 19,2 (2C, CH₂), 12,9 (2C, CH₂); MALDI-TOF[m/z]: 172,2 (M+H⁺).

### Rezepturen:

### Anwendungsstechnische Überprüfung:

### Verwendete Haare:

Euro-Haar (Haar von Europäern), naturbelassen.

### Vorschädigung der Haare:

Je 1 x Dauerwellen und Bleichen mit handelsüblichen Produkten.

### Behandlung der geschädigten Haare:

Die geschädigten Haare werden mit den Beispielformulierungen behandelt.

**Tabelle 1:**

| | Haarspülung A | Haarspülung B (nicht erfindungsgemäß) |
|---|---|---|
| Alkylguanidinium-Verbindung [Gew.-%] | 2,0 | - |
| Ceteareth-25 [Gew.-%] | 0,5 | 0,5 |
| Cetearylalcohol [Gew.-%] | 2,0 | 2,0 |
| HCl | ad pH = 5 | ad pH = 5 |
| Wasser [Gew.-%] | ad 100 | ad 100 |

Die Verbesserung der mechanischen Widerstandskraft geschädigter Haare durch Alkylguanidinium-Verbindungen wird durch paarweisen Vergleich von Einzelhaaren vor und nach Behandlung mit der Testformulierung ermittelt.

Für jede Testformulierung werden 30 feuchte Einzelhaare vermessen. Die Versuchsdurchführung sieht folgendermaßen aus:
1. Die vorgeschädigten Haare werden mit Wasser angefeuchtet und mit dem Vollautomat Mtt670 der Firma DiaStron die Kraft gemessen, die zur 15 %igen Dehnung erforderlich ist.
2. Die Haare werden zur Erholung mind. 2 h in einem Wasserbad getaucht.
3. Danach werden die Haare 30 min in der Testformulierung getaucht und anschließend jedes Haar ca. 7 s unter fließendem Wasser abgespült.
4. Man lässt die Haare über Nacht (= 12 h) an der Luft trocken.
5. Die Kraft, die erforderlich ist, um die behandelten Haare um 15 % zu dehnen, wird wieder wie oben beschrieben, nach Anfeuchten mit Wasser, gemessen.
6. Die Differenz der 15 % Dehnungskräfte vor und nach Behandlung mit der Testformulierung wird berechnet und als Maß für die Verbesserung der mechanischen Widerstandskraft der geschädigten Haare durch die Alkylguanidin-Verbindungen herangezogen.

Zur statistischen Beurteilung der Messwerte werden der t-Test zum paarweisen Vergleich sowohl der Messwerte vor und nach der Behandlung als auch der Messwerte der behandelten Haare mit dem Ergebnis des placebo Ergebnisses herangezogen. Damit lässt sich eine Aussage über die statistische Sicherheit der Messwerte treffen (hierzu siehe auch: R.E. Kaiser, J.A. Mühlbauer, "Elementare Tests zur Beurteilung von Meßdaten", B.I. Wissenschaftsverlag, Mannheim 1983). Man geht bei einer statistischen Sicherheit von > 95 % davon aus, dass ein signifikanter Unterschied besteht, > 99 % bedeutet, dass sich die Messwerte hoch signifikant unterscheiden, > 99,9, dass sie sich höchstsignifikant unterscheiden.

**Tabelle 2: Bestimmung der Kraft zur Dehnung einzelner Haare um 15 % vor und nach deren Behandlung (mindestens 30 unabhängige Messungen) :**

| | | | | |
|---|---|---|---|---|
| Testformulierungen nach Tab. 1 mit erfindungsgemäßer Verbindung Formel (I) mit R¹ = H vorliegend als Essigsäuresalz | Kraft 15 %_{nach} Kraft 15 %ᵥₒᵣ [mN] | σ | Paarweiser t-Test [%] | t-Test: Vergleich mit Mittelwert Placebo [%] |
| HSP B | 0,49 | 2,41 | 72,9 | |
| HSP A mit R² = C₄ | 1,36 | 1,62 | 100,0 | 89,0 |
| HSP A mit R² = C₆ | 1,72 | 1,61 | 100,0 | 97,8 |
| HSP A mit R² = C₈ | 3,51 | 1,64 | 100,0 | 100,0 |
| HSP A mit R²= C₁₀ | 3,00 | 1,83 | 100,0 | 100,0 |
| HSP A mit R² = C₁₂ | 3,30 | 1,64 | 100,0 | 100,0 |
| HSP A mit R² = C₁₆ | 1,89 | 1,33 | 100,0 | 99,3 |
| HSP A mit R² = C₁₈ | 1,80 | 1,45 | 100,0 | 98,7 |
| HSP A mit R² = C₂₂ | 0,94 | 2,21 | 97,3 | 54,0 |

HSP: Haarspülung
Kraft 15 %_{nach}: 15 % Dehnungskraft nach Behandlung mit der Testformulierung.
Kraft 15 %ᵥₒᵣ: 15 % Dehnungskraft vor Behandlung mit der Testformulierung.
σ: Standardabweichung

Die Alkylguanidin-Verbindungen sind also in der Lage, die 15 % Dehnungskräfte geschädigter Haare hochsignifikant zu erhöhen d.h. sie können die mechanische Widerstandskraft geschädigter Haare überraschend deutlich verbessern.

### Schutz der Haare vor Schädigung durch chemische Mittel:

Je stärker geschädigt Haare sind, z.B. durch Dauerwellen oder Bleichen, desto höher ist die Konzentration wasserlöslicher Proteinfragmente/Peptide im Haar, die durch Extraktion mit Wasser oder Tensiden extrahiert werden kann. Die Extraktion mit Tensiden simuliert das Waschen der Haare durch ein Shampoo. Auch bei der Haarwäsche verlieren geschädigte Haare mehr Peptide als gesundes Haar.

Zur Verdeutlichung wird das Haar vorgeschädigt. Dazu werden die Haare je 1 x dauergewellt und gebleicht.

**Tabelle 3: Testformulierung einer Haarspülung mit 2 % Wirkstoff:**

| | |
|---|---|
| TEGINACID® C (Emulgator) | 0,5 % |
| TEGO® Alkanol 1618 (Konsistenzgeber) | 2,0 % |
| Alkylguanidin-Verbindung | 2,0 % AS |
| Konservierungsmittel (CA24) | q.s. |
| HCl | pH = 5,0 |
| Wasser | ad 100,0 % |

Die vorgeschädigten Haarsträhnen (Gewicht: ca. 4 g) werden in die Testformulierung getaucht (Einwirkzeit: 30 min) und anschließend 1 min unter fließendem Leitungswasser (T = 35 °C) ausgespült. Dann lässt man sie 12 Stunden an der Luft trocknen.

### Bestimmung des Proteinverlustes:

ca. 0,5 g Haar + ca. 15 ml Na-Laurylethersulfat (8 %) werden 3 h bei Raumtemperatur geschüttelt. Anschließend wird die Lösung dekantiert und zentrifugiert. Dann wird die Protein-Konzentration der Lösung photometrisch durch Messung der UV-Absorption bei 295 nm bestimmt. Als Standard dient ein Keratinhydrolysat (M = 1000, Promois WK-H, Seiwa Kasei).

**Tabelle 4: Bestimmung des Proteinverlustes bezogen auf 1 g Haar (mindestens drei unabhängige Messungen):**

| | | |
|---|---|---|
| Formulierung nach Tab. 3 mit erfindungsgemäßer Verbindung Formel (I) mit R¹ = H vorliegend als Essigsäuresalz | Protein-Verlust [mg/g Haar] | σ |
| virgin | 13,7 | 0,39 |
| geschädigt | 35,0 | 0,87 |
| placebo | 28,8 | 0,62 |
| R² = C₄ | 20,3 | 0,12 |
| R² = C₆ | 26,0 | 1,25 |
| R² = C₈ | 26,8 | 0,43 |
| R² = C₁₀ | 26,4 | 0,79 |
| R² = C₁₂ | 23,5 | 0,67 |
| R² = C₁₆ | 25,9 | 0,79 |
| R² = C₁₈ | 29,8 | 0,54 |
| R² = C₂₂ | 28,9 | 0,84 |

| | | |
|---|---|---|
| σ = Standardabweichung | | |

Man erkennt, dass die Alkylguanidinium-Verbindungen den Protein-Verlust der geschädigten Haare reduzieren, insbesondere die kurzkettigen Verbindungen zeigen hier eine sehr gute Schutzwirkung.

## Patentansprüche

1. Verwendung von Alkylguanidinverbindungen der allgemeinen Formeln (I) und/oder (II) 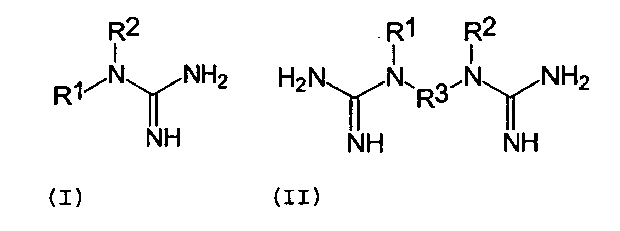 und/oder deren Salze oder Hydrate, in denen
R¹, R² = a) unabhängig voneinander H, ein gegebenenfalls verzweigter, gegebenenfalls Doppelbindungen enthaltender Kohlenwasserstoffrest, Hydroxyalkyl-, Alkyloxy-, Carboxyalkylreste, mit 2 bis 30 C-Atomen, vorzugsweise 4 bis 22, insbesondere 8 bis 12 C-Atomen, oder
R¹, R² = b) wobei die Reste auch ali- oder heterocyclische Komponenten, gesättigt, ungesättigt oder aromatisch, mit einer Ringgröße von 3 bis 10 Atomen, vorzugsweise von 4 bis 6 Atomen, besitzen können, welche weitere, gesättigte oder ungesättigte Kohlenwasserstoffsubstituenten mit 1 bis 30 C-Atomen, vorzugsweise 4 bis 22 C-Atomen, tragen können, oder
R¹, R² = c) Alkylamidoalkylen- oder Alkylesteralkylenreste, die weitere unter a) und b) genannte Strukturelemente enthalten können, und die Strukturelemente a), b), c) untereinander und miteinander kombiniert werden können,
R³ Alkylen, gegebenenfalls verzweigter, gegebenenfalls Doppelbindungen oder ali- oder heterocyclische Komponenten, gesättigt, ungesättigt oder aromatisch, mit einer Ringgröße von 3 bis 10 Atomen, vorzugsweise mit einer Ringgröße von 4 bis 6 Atomen, enthaltender Kohlenwasserstoffrest mit 1 bis 30 C-Atomen, vorzugsweise 4 bis 22 C-Atomen oder in der R¹ und R² in dem Element -N(R¹)-R³-(R²)N- einen 5- bis 8-gliedrigen Ring bilden kann, enthalten
als Wirksubstanzen zur Herstellung eines Haarbehandlungsmittels/Haarnachbehandlungsmittels zur Verhütung von Schäden durch chemische Behandlungsmittel und zur Reparatur bereits geschädigter Haare.

2. Verwendung von Alkylguanidinverbindungen gemäß Anspruch 1, Formel (I) und/oder deren Salze, **dadurch gekennzeichnet, dass** mindestens einer der Reste R¹, R² nicht Wasserstoff ist.

3. Verwendung von Alkylguanidinverbindungen gemäß Anspruch 1, Formel (II) und/oder deren Salze, **dadurch gekennzeichnet, dass** R³ Kohlenwasserstoffreste, vorzugsweise Alkylenreste mit 4 bis 18 C-Atomen sind und R¹ und R² die oben angegebene Bedeutung haben.

4. Verwendung von Alkylguanidinverbindungen gemäß den Ansprüchen 1 bis 3, **dadurch gekennzeichnet, dass** sie als Salze mindestens eine der Säuren ausgewählt aus der Gruppe Ameisensäure, Essigsäure, Propionsäure, Heptansäure, Caprylsäure, Nonansäure, Caprinsäure, Undecansäure, Laurinsäure, Myristinsäure, Palmitinsäure, Stearinsäure, Arachinsäure, Behensäure, Cyclopentancarbonsäure, Cyclohexancarbonsäure, Acrylsäure, Methacrylsäure, Vinylessigsäure, Crotonsäure, 2-/3-/4-Pentensäure, 2-/3-/4-/5-Hexensäure, Lauroleinsäure, Myristoleinsäure, Palmitoleinsäure, Ölsäure, Gadoleinsäure, Sorbinsäure, Linolsäure, Linolensäure, Pivalinsäure, Ethoxyessigsäure, Phenylessigsäure, Milchsäure, 2-Ethylhexansäure, Oxalsäure, Glycolsäure, Äpfelsäure, Malonsäure, Bernsteinsäure, Weinsäure, Glutarsäure, Zitronensäure, Adipinsäure, Pimelinsäure, Korksäure, Azelainsäure, Sebacinsäure, Benzoesäure, o-/m-/p-Tolylsäure, Salicylsäure, 3-/4-Hydroxybenzoesäure, Phthalsäuren, oder deren ganz oder teilweise hydrierten Derivate wie Hexahydro- oder Tetrahydrophthalsäure, Phosphorsäure, Salzsäure, Schwefelsäure und deren Gemische, insbesondere Milchsäure, Weinsäure, Essigsäure und Salzsäure mitverwendet werden.

5. Verwendung von Alkylguanidinverbindungen gemäß Anspruch 1, **dadurch gekennzeichnet, dass** sie 0,01 bis 10,0 Gew.-% der Verbindungen der allgemeinen Formeln (I) und/oder (II) und/oder deren Salze und/oder deren Hydrate enthalten.

6. Verwendung von Alkylguanidinverbindungen gemäß den Ansprüchen 1 bis 5, enthaltend 0 bis 10 Gew.-% eines oder mehrerer Emulgatoren, 0 bis 10 Gew.-% eines oder mehrerer Konsistenzgeber, 0 bis 8 Gew.-% eines oder mehrerer, vorzugsweise kationischer Tenside, 0 bis 20 Gew.-% eines oder mehrerer kosmetischer Öle oder Emollients sowie übliche Hilfs- und Zusatzstoffe in üblichen Konzentrationen, **dadurch gekennzeichnet, dass** sie 0,01 bis 10,0 Gew.-% der Verbindungen der allgemeinen Formeln (I) und/oder (II) und/oder deren Salze und/oder deren Hydrate gemäß den Ansprüchen 1 bis 4 enthalten.

7. Verwendung von Alkylguanidinverbindungen gemäß Anspruch 6, enthaltend zusätzlich ein oder mehrere haarkosmetische Wirkstoffe ausgesucht aus der Gruppe der Proteinhydrolysate pflanzlichen oder tierischen Ursprungs auf Basis Keratin, Collagen, Elastin, Weizen, Reis, Soja, Milch, Seide, Mais; Antischuppenwirkstoffe wie Piroctone Olamine, Zink Omadine und Climbazol, Sebostatika; Vitamine, Panthenol, Pyrrolidoncarbonsäure, Bisabolol, Pflanzenextrakte, Kreatin, Ceramide.

## Claims

1. Use of alkylguanidine compounds of the general formulae (I) and/or (II) 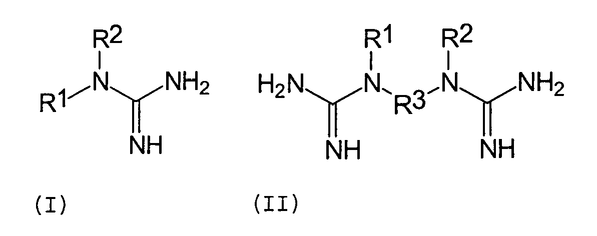 and/or their salts or hydrates, in which
R¹, R² a) independently of one another are H, an optionally branched hydrocarbon radical optionally containing double bonds, hydroxyalkyl, alkyloxy, carboxyalkyl radicals, having 2 to 30 C atoms, preferably 4 to 22, in particular 8 to 12, C atoms, or
R¹, R² b) where the radicals can also have alicyclic or heterocyclic components, saturated, unsaturated or aromatic, having a ring size of 3 to 10 atoms, preferably from 4 to 6 atoms, which can carry further, saturated or unsaturated hydrocarbon substituents having 1 to 30 C atoms, preferably 4 to 22 C atoms, or
R¹, R² c) alkylamidoalkylene or alkyl ester alkylene radicals, which can contain further structural elements mentioned under a) and b),
and the structural elements a), b), c) can be combined among one another and with one another,
R³ is alkylene, an optionally branched hydrocarbon radical having 1 to 30 C atoms, preferably 4 to 22 C atoms, optionally containing double bonds or alicyclic or heterocyclic components, which is saturated, unsaturated or aromatic, having a ring size of 3 to 10 atoms, preferably having a ring size of 4 to 6 atoms, or in which R¹ and R² in the element -N(R¹)-R³-(R²)N- can form a 5- to 8-membered ring,
as active substances for the production of a hair treatment composition/hair aftertreatment composition for the prevention of damage by chemical treatment compositions and for the repair of already damaged hair.

2. Use of alkylguanidine compounds according to Claim 1, formula (I) and/or their salts, **characterized in that** at least one of the radicals R¹, R² is not hydrogen.

3. Use of alkylguanidine compounds according to Claim 1, formula (II) and/or their salts, **characterized in that** R³ is hydrocarbon radicals, preferably alkylene radicals having 4 to 18 C atoms and R¹ and R² have the meaning indicated above.

4. Use of alkylquanidine compounds according to Claims 1 to 3, **characterized in that**, as salts, at least one of the acids selected from the group consisting of formic acid, acetic acid, propionic acid, heptanoic acid, caprylic acid, nonanoic acid, capric acid, undecanoic acid, lauric acid, myristic acid, palmitic acid, stearic acid, arachic acid, behenic acid, cyclopentanecarboxylic acid, cyclohexanecarboxylic acid, acrylic acid, methacrylic acid, vinylacetic acid, crotonic acid, 2-/3-/4-pentenoic acid, 2-/3-/4-/5-hexenoic acid, lauroleic acid, myristoleic acid, palmitoleic acid, oleic acid, gadoleic acid, sorbic acid, linoleic acid, linolenic acid, pivalic acid, ethoxyacetic acid, phenylacetic acid, lactic acid, 2-ethylhexanoic acid, oxalic acid, glycolic acid, malic acid, malonic acid, succinic acid, tartaric acid, glutaric acid, citric acid, adipic acid, pimelic acid, suberic acid, azelaic acid, sebacic acid, benzoic acid, o-/m-/p-toluic acid, salicylic acid, 3-/4-hydroxybenzoic acid, phthalic acids, or their completely or partly hydrogenated derivatives such as hexahydro- or tetrahydrophthalic acid, carbonic acid, phosphoric acid, hydrochloric acid, sulfuric acid and their mixtures, in particular lactic acid, tartaric acid, acetic acid and hydrochloric acid are additionally used.

5. Use of alkylguanidine compounds according to Claim 1, which contain 0.01 to 10.0% by weight of the compounds of the general formulae (I) and/or (II) and/or their salts and/or their hydrates.

6. Use of alkylguanidine compounds according to Claims 1 to 5, comprising 0 to 10% by weight of one or more emulsifiers, 0 to 10% by weight of one or more consistency-imparting agents, 0 to 8% by weight of one or more, preferably cationic, surfactants, 0 to 20% by weight of one or more cosmetic oils or emollients, and customary excipients and additives in customary concentrations, which contain 0.01 to 10.0% by weight of the compounds of the general formulae (I) and/or (II) and/or their salts and/or their hydrates as set forth in Claims 1 to 4.

7. Use of alkylguanidine compounds according to Claim 6, additionally comprising one or more hair cosmetic active ingredients selected from the group consisting of the protein hydrolysates of vegetable or animal origin based on keratin, collagen, elastin, wheat, rice, soybeans, milk, silk, corn; antidandruff active ingredients such as piroctone olamine, zinc omadine and climbazole, sebostatics; vitamins, panthenol, pyrrolidonecarboxylic acid, bisabolol, plant extracts, creatine, ceramides.

## Revendications

1. Utilisation de composés d'alkylguanidine des formules générales (I) et/ou (II) 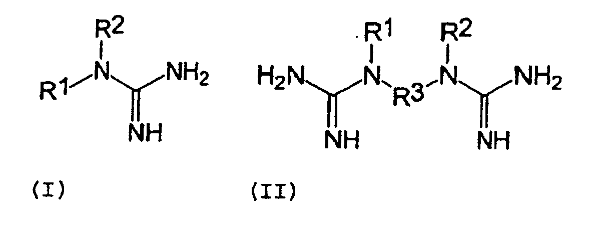 et/ou leurs sels ou hydrates, dans lesquelles
R¹, R² = a) indépendamment l'un de l'autre H, un radical hydrocarboné le cas échéant ramifié, contenant le cas échéant des doubles liaisons, des radicaux hydroxyalkyle, alkyloxy, carboxyalkyle comprenant 2 à 30 atomes de carbone, de préférence 4 à 22, en particulier 8 à 12 atomes de carbone, ou
R¹, R² = b) les radicaux pouvant également présenter des composants alicycliques ou hétérocycliques, saturés, insaturés ou aromatiques, présentant une taille de cycle de 3 à 10 atomes, de préférence de 4 à 6 atomes, qui peuvent porter d'autres substituants hydrocarbonés saturés ou insaturés comprenant 1 à 30 atomes de carbone, de préférence 4 à 22 atomes de carbone, ou
R¹, R² = c) des radicaux alkylamidoalkylène ou alkylesteralkylène, qui peuvent contenir d'autres éléments de structure mentionnés aux points a) et b), et les éléments de structure a), b), c) peuvent être combinés ensemble et les uns avec les autres,
R³ représente alkylène, un radical hydrocarboné le cas échéant ramifié, contenant le cas échéant des doubles liaisons ou des composants alicycliques ou hétérocycliques, saturés, insaturés, ou aromatiques, présentant une taille de cycle de 3 à 10 atomes, de préférence une taille de cycle de 4 à 6 atomes, comprenant 1 à 30 atomes de carbone, de préférence 4 à 22 atomes de carbone ou dans lequel R¹ et R² dans l'élément -N(R¹)-R³-(R²)N- pouvant former un cycle de 5 à 8 chaînons,
comme substances actives pour la préparation d'un agent de traitement/post-traitement des cheveux pour empêcher des dégradations par des agents de traitement chimiques et pour la réparation de cheveux déjà dégradés.

2. Utilisation de composés d'alkylguanidine selon la revendication 1, formule (I) et/ou leurs sels, **caractérisée en ce qu'**au moins un des radicaux R¹, R² n'est pas de l'hydrogène.

3. Utilisation de composés d'alkylguanidine selon la revendication 1, formule (II) et/ou leurs sels, **caractérisée en ce que** R³ représente des radicaux hydrocarbonés, de préférence des radicaux alkylène comprenant 4 à 18 atomes de carbone et R¹ et R² présentent la signification susmentionnée.

4. Utilisation de composés d'alkylguanidine selon les revendications 1 à 3, **caractérisée en ce que**, comme sels, au moins un des acides choisis dans le groupe constitué par l'acide formique, l'acide acétique, l'acide propionique, l'acide heptanoïque, l'acide caprylique, l'acide nonanoïque, l'acide caprique, l'acide undécanoïque, l'acide laurique, l'acide myristique, l'acide palmitique, l'acide stéarique, l'acide arachidique, l'acide béhénique, l'acide cyclopentanecarboxylique, l'acide cyclohexanecarboxylique, l'acide acrylique, l'acide méthacrylique, l'acide vinylacétique, l'acide crotonique, l'acide 2-/3-/4-penténoïque, l'acide 2-/3-/4-/5-hexénoïque, l'acide lauroléique, l'acide myristoléique, l'acide palmitoléique, l'acide oléique, l'acide gadoléique, l'acide sorbique, l'acide linoléique, l'acide linolénique, l'acide pivalique, l'acide éthoxyacétique, l'acide phénylacétique, l'acide lactique, l'acide 2-éthylhexanoïque, l'acide oxalique, l'acide glycolique, l'acide malique, l'acide malonique, l'acide succinique, l'acide tartrique, l'acide glutarique, l'acide citrique, l'acide adipique, l'acide pimélique, l'acide subérique, l'acide azélaïque, l'acide sébacique, l'acide benzoïque, l'acide o-/m-/p-tolylique, l'acide salicylique, l'acide 3-/4-hydroxybenzoïque, les acides phtaliques ou leurs dérivés totalement ou partiellement hydrogénés, tels que l'acide hexahydrophtalique ou tétrahydrophtalique, l'acide carbonique, l'acide phosphorique, l'acide chlorhydrique, l'acide sulfurique et leurs mélanges, en particulier l'acide lactique, l'acide tartrique, l'acide acétique et l'acide chlorhydrique sont conjointement utilisés.

5. Utilisation de composés d'alkylguanidine selon la revendication 1, **caractérisée en ce qu'**ils contiennent 0,01 à 10,0% en poids des composés des formules générales (I) et/ou (II) et/ou leurs sels et/ou leurs hydrates.

6. Utilisation de composés d'alkylguanidine selon les revendications 1 à 5, contenant 0 à 10% en poids d'un ou de plusieurs émulsifiants, 0 à 10% en poids d'un ou de plusieurs agents conférant une consistance, 0 à 8% en poids d'un ou de plusieurs agents tensioactifs de préférence cationiques, 0 à 20% en poids d'un(e) ou de plusieurs huiles ou émollients cosmétiques ainsi que des adjuvants et additifs usuels en des concentrations usuelles, **caractérisée en ce qu'**ils contiennent 0,01 à 10,0% en poids des composés des formules générales (I) et/ou (II) et/ou leurs sels et/ou leurs hydrates selon les revendications 1 à 4.

7. Utilisation de composés d'alkylguanidine selon la revendication 6, contenant en outre une ou plusieurs substances actives cosmétiques capillaires du groupe des hydrolysats de protéines d'origine végétale ou animale, à base de kératine, collagène, élastine, blé, riz, soja, lait, soie, maïs ; des substances actives anti-pelliculaires, telles que les piroctones, les olamines, les omadines de zinc et le climbazol, les sébostatiques ; des vitamines, du panthénol, de l'acide pyrrolidonecarboxylique, du bisabolol, des extraits végétaux, de la créatine, des céramides.
